# EUROPÄISCHE PATENTANMELDUNG

(11) **EP 3 053 539 A1**
(43) Veröffentlichungstag der Anmeldung: **10.08.2016**
(21) Anmeldenummer: 15154144.8
(22) Anmeldetag: 06.02.2015
(51) Int. Cl.: A61B 18/20

(54) **Laser zur Bestrahlung der Haut**

(71) Anmelder: Fatemi, Afschin, 40474 Düsseldorf (DE)
(72) Erfinder: Fatemi, Afschin, 40474 Düsseldorf (DE)
(74) Vertreter: Roth, Andy Stefan

(57) **Zusammenfassung**

Die Erfindung betrifft eine Vorrichtung zur Bestrahlung einer Zielstruktur (7), die sich innerhalb von menschlicher oder tierischer Haut (11) oder Gewebe befindet. Die Bestrahlungsvorrichtung (1) verfügt über wenigstens eine Laserlichtquelle (2) und zumindest ein Lichtlenkmittel (3), die derart ausgeführt sind, dass Laserstrahlung über unterschiedliche Strahlengänge in die Zielstruktur (7) gelangt und aufgrund eines Energieeintrags in die Zielstruktur (7) zumindest bereichsweise eine Eigenschaftsveränderung der Zielstruktur (7) erfolgt.

Die beschriebene technische Lösung zeichnet sich dadurch aus, dass eine Steuerung (4) vorgesehen ist, so dass die Eigenschaftsveränderung gezielt in einem Bereich (8) mit räumlicher Ausdehnung realisierbar ist.

## Beschreibung

Die Erfindung betrifft eine Vorrichtung zur Bestrahlung einer Zielstruktur, die sich innerhalb oder unterhalb von menschlicher oder tierischer Haut oder Gewebe befindet. Die Vorrichtung verfügt hierbei über wenigstens eine Laserlichtquelle und zumindest ein Lichtlenkmittel, die derart ausgeführt sind, dass Laserstrahlung über unterschiedliche Strahlengänge in die Zielstruktur gelangt, wobei aufgrund eines durch die Bestrahlung bedingten Energieeintrags zumindest bereichsweise eine Eigenschaftsveränderung der Zielstruktur erfolgt.

Die von einem Laser ausgesandten Strahlen zeichnen sich vor allem durch ihre hohe Intensität, den sehr engen Frequenzbereich der Strahlung und damit einhergehende targetspezifischen Selektivität, eine scharfe Bündelung des Strahls sowie die große Kohärenzlänge aus. Neben vielen bekannten Anwendungen im technischen Bereich, findet der Laser auch vielfach in der Medizintechnik Anwendung. So werden beispielsweise in der Dermatologie mit Hilfe von Laserstrahlen Schnitte und Verödungen durchgeführt. Ebenso können Blutgefäße durch Laser bestimmter Wellenlänge koaguliert werden und Pigmentflecken mit Hilfe ablatierender, bzw. sogenannter schälender Laser abgetragen oder selektiv zerstört werden. Ferner werden subkutane Pigmente mit Hilfe einer ultrakurzgepulsten Laserquelle zerstört und damit entfernt, ohne die Hautoberfläche nachhaltig zu verletzen, oder mit Hilfe von langgepulsten Lasern Haarwurzeln durch Epilation dauerhaft zerstört. Darüber hinaus werden Laser teilweise zur gezielten Behandlung entzündlicher Hauterkrankungen, wie etwa der Psoriasis (Schuppenflechte), eingesetzt oder es werden oberflächliche Unebenheiten der Haut, wie Knötchen oder Fältchen, zur kosmetischen Verbesserung des Hautbildes geglättet (Resurfacing). Gemäß einer weiteren Anwendung in der Dermatologie werden Laser verwendet, um selektiv dermale Anteile zu erwärmen und so den Kollagenaufbau zu fördern und die Haut in diesem Bereich zu straffen (Subsurfacing).

Je nach der Art des verwendeten Lasers kommt es zu unterschiedlichen Wechselwirkungen zwischen der Haut bzw. dem Gewebe und dem vom Laser ausgesendeten Licht. Die Art der Wechselwirkung hängt einerseits von den optischen Eigenschaften der Haut bzw. des Gewebes, insbesondere dem Streu- und dem Absorptionskoeffizienten sowie der Dichte, und andererseits von den physikalischen Parametern des Laserlichts, insbesondere der Wellenlänge, der Energiedichte, der Wiederholrate, der Bestrahlungsdauer sowie der Spotgröße, ab.

Die Laser-Gewebe-Wechselwirkungen werden in verschiedene Mechanismen eingeteilt. Hierzu gehören die photo-thermale, photo-mechanische bzw. photo-akustische und die photo-chemische Wechselwirkung. Weiterhin wird teilweise noch das Phänomen der photo-vermittelten Ablation, die sogenannte Photoablation, genutzt. Unter diesen Effekten ist die am meisten quantifizierbare und zumeist beobachtende Reaktion der photo-thermische Effekt, der durch die Einbringung hoher Energien und die dadurch verursachte Verdampfung von Wasser im Gewebe erreicht wird. Je nach Beschaffenheit des bestrahlten Mediums und der entsprechenden Struktur sowie der Bestrahlungsparameter treten die verschiedenen Effekte unterschiedlich stark auf, wobei angenommen wird, dass die absorbierte Energie und die Bestrahlungszeit die Gewebewirkung zum größten Teil beeinflussen. Die entsprechenden Wechselwirkungen werden im Detail in "C. Raulin, S. Kasei (Hrsg.), Lasertherapie der Haut, Springer-Verlag Berlin, Heidelberg 2013" beschrieben.

In diesem Zusammenhang wird in der US 2011/0313408 A1 ein Laser zur Behandlung der Haut beschrieben. Wesentlich an der beschriebenen technischen Lösung ist, dass für die Bestrahlung ein erster Laserstrahl mit langer Pulsdauer und ein zweiter Laserstrahl mit kurzer Pulsdauer unter gleichzeitigem Einsatz eines Kühlelements zum Kühlen eines Oberflächenbereichs der Haut verwendet werden. Mit dem ersten Laserstrahl wird ein Volumen von Hautgewebe unterhalb des gekühlten Oberflächenbereichs erwärmt, um das Hautgewebe noch unterhalb der Hautgewebeschädigungsschwelle zu modifizieren. Der zweite Laserstrahl wird in eine Vielzahl von getrennten Laserstrahlen mit Hilfe von getrennten optischen Fasern aufgeteilt und über getrennte Wege durch das Hautgewebe in das vorerwärmte Hautgewebe gelenkt, um hier in gezielt ausgewählten, kleinen Volumina mechanische Beschädigungen hervorzurufen.

Ferner ist aus der US 2007/0239147 A1 ein System zur Behandlung von dermatologischen Erkrankungen bekannt, das auf der thermischen Schädigung einer Zielstruktur beruht. Hierbei wird gezielt ein Strahlenbündel auf einen Zielpunkt auf der Hautoberfläche gerichtet, um die Zielstruktur im Gewebe unterhalb dieser Zielposition zu schädigen. Insbesondere sind Lichtlenkmittel vorgesehen, mit denen ein zweiter Lichtstrahl auf die gleiche Zielposition gelenkt wird, um ein anderes Gewebevolumen unterhalb des Zielortes thermisch zu schädigen.

Aus der US 7,066,929 B1 ist ferner eine selektive Photothermolyse bekannt, mit der subkutanes Gewebe durch Verwendung einer Mehrzahl von Strahlen von schmalbandigen, elektromagnetischen Wellen zerstört wird. Da jeder der Strahlen nicht über die ausreichende Energie verfügt, um das Gewebe auf die benötigte Temperatur zu erwärmen und durch Übererhitzung zu zerstören, werden die einzelnen Strahlen am Zielpunkt derart überlappt, so dass ausreichende Wärme erzeugt wird, um das Zielgewebe zu zerstören. Gemäß der beschriebenen technischen Lösung ist hierfür eine Strahllenkeinrichtung vorgesehen, die die einzelnen Lichtstrahlen derart lenkt, dass jeder der Strahlen beim Verlassen der Strahlungsvorrichtung in einem anderen Winkel austritt. Mit Hilfe einer geeigneten Steuerung des Lasers werden die unterschiedlichen einzelnen Strahlen schließlich in einem Punkt fokussiert.

Im Weiteren beschreibt die WO 2013/156421 A1 ebenfalls eine Vorrichtung zur Behandlung von Haut- und/oder Gewebeschichten mit Hilfe von Laserlicht. Wesentlich an der beschrieben Vorrichtung ist, dass eine Vielzahl von Laserlichtquellen verwendet wird, um gleichzeitig oder abwechselnd eine Zielstruktur aus unterschiedlichen Richtungen zu bestrahlen. Die Laserlichtquellen und/oder geeignete Lichtlenkmittel sind derart angeordnet, dass das Laserlicht auf unterschiedlichen Wegen in die zu behandelnde Haut- oder Gewebeschicht und im Bereich der Zielstruktur punktuell fokussiert wird.

Problematisch an den bekannten und derzeit zum Einsatz kommenden technischen Lösungen zur Behandlung der Haut ist oftmals, dass der Hautbereich, auf den die Laserstrahlung auftrifft, um von dort aus weiter in die Haut bzw. das subkutane Gewebe einzudringen, geschädigt wird. Derartige Schädigungen sind regelmäßig nicht erwünscht und können teilweise zu nicht unerheblichen Komplikationen führen. Weiterhin stellt es in vielen Fällen ein erhebliches Problem dar, gezielt größere, insbesondere subkutan in vergleichsweise großer Tiefe gelegene Zielstrukturen zu zerstören, ohne dass es zu Schädigungen von angrenzenden Gewebebereichen, Drüsen oder anderen Teilen der Haut oder des Gewebes kommt. Dies ist vor allem darauf zurückzuführen, dass es mit den bekannten technischen Lösungen nicht in ausreichendem Maße möglich ist, einen ausreichenden und trotzdem lokal begrenzten Energieeintrag in eine ausgedehnte Zielstruktur innerhalb der Haut oder des subkutanen Gewebes zu realisieren.

Ausgehend von dem aus dem Stand der Technik bekannten technischen Lösungen sowie den zuvor geschilderten Problemen liegt der Erfindung die Aufgabe zu Grunde, eine Vorrichtung zur Behandlung der Haut eines Menschen oder eines Tieres mittels eines Lasers derart weiterzubilden, dass der benötigte Energieeintrag zumindest nahezu ausschließlich in die zu schädigende Zielstruktur erfolgt. Hierbei soll insbesondere eine Schädigung der Epidermis der Haut, auf die Laserstrahlung auftritt, vermieden werden und insgesamt der Energieeintrag in die Haut bzw. das Gewebe auf das jeweils benötigte Mindestmaß reduziert werden. Das beschriebene System soll sich weiterhin auf vergleichsweise einfache Weise in ein kompaktes Gerät integrieren lassen, und einen wirtschaftlichen Einsatz im täglichen Betrieb ermöglichen. Weiterhin soll das anzugebende System derart ausgeführt werden, dass ein flexibler Einsatz, insbesondere die Durchführung einer Vielzahl von unterschiedlichen Behandlungen der Haut und des darunter liegenden Gewebes möglich ist.

Die zuvor beschriebene Aufgabe wird mit einer Vorrichtung gemäß Anspruch 1 gelöst. Ein geeignetes Verfahren zur Steuerung einer Laserlichtquelle ist im Anspruch 12 sowie eine auf der Erfindung beruhende Verwendung eines entsprechend erzeugten Laserstrahls ist in Anspruch 13 angegeben. Vorteilhafte Ausführungsformen der Erfindung sind in den abhängigen Ansprüchen angegeben und werden in der folgenden Beschreibung unter teilweiser Bezugnahme auf die Figuren näher erläutert.

Gemäß der Erfindung ist eine Vorrichtung zur Bestrahlung einer Zielstruktur, die sich innerhalb von menschlicher oder tierischer Haut oder Gewebe befindet, mit wenigstens einer Laserlichtquelle und zumindest einem Lichtlenkmittel, die derart ausgeführt sind, dass Laserstrahlung über unterschiedliche Strahlengänge in die Zielstruktur gelangt und aufgrund eines Energieeintrags in die Zielstruktur zumindest bereichsweise eine Eigenschaftsveränderung der Zielstruktur erfolgt, derart weitergebildet worden, dass eine Steuerung vorgesehen ist, so dass die Eigenschaftsveränderung gezielt in einem Bereich mit räumlicher Ausdehnung in der Haut oder dem Gewebe realisierbar ist. Unter einem Lichtlenkelement im Sinne der Erfindung wird jedes Mittel verstanden, dass den Laserstrahl in Bezug auf seinen Strahlungszustand und/oder seinen Strahlengang beeinflusst. Insbesondere kann es sich hierbei sowohl um technische Einrichtungen handeln, die ein gezieltes, bedarfsgerechtes Ein- und Ausschalten, insbesondere ein Pulsen, der wenigstens einen Laserlichtquelle ermöglichen als auch um optische Elemente, die die Ausbreitung des Laserstrahls und/oder seine optischen Eigenschaften beeinflussen.

Wesentlich an der erfindungsgemäßen technischen Lösung ist somit, dass die wenigstens eine Laserlichtquelle und/oder das Lichtlenkmittel derart angesteuert werden, dass mit Hilfe mehrerer Laserstrahlen gezielt ein dreidimensionaler Bestrahlungsbereich bzw. eine räumliche Struktur innerhalb der Haut oder des subkutanen Gewebes auf den jeweils benötigten Temperaturwert erwärmt und gezielt die Eigenschaft verändert, insbesondere das bestrahlte Gewebe koaguliert oder zerstört werden kann. Ein ganz wesentlicher Aspekt hierbei ist, dass die Laserstrahlung nicht auf einen Punkt fokussiert wird, sondern gezielt ein Bereich mit räumlicher Ausdehnung mit Laserstrahlung bestrahlt wird. Dies kann wahlweise erreicht werden, indem ein Laser verwendet wird, der bewegbar ausgeführt sein kann, und die jeweilige Laserstrahlung gepulst und/oder umgelenkt wird oder, dass wenigstens zwei Laserstrahlen verwendet werden, und die Strahlung jeweils wiederum auf geeignete Weise auf die Haut eines Patienten auftrifft.

Gemäß einer ersten Ausführungsform der Erfindung ist die Laserlichtquelle und/oder das Lichtlenkelement derart ansteuerbar, dass der Bereich, in dem gezielt eine Eigenschaftsveränderung realisiert werden soll, linsenförmig ausgebildet ist. Die Ansteuerung der Laserlichtquelle und/oder des Lichtlenkmittels kann hierbei derart ausgeführt werden, dass die Haupterstreckungsrichtung der Linse zumindest annährend parallel zur Hautoberfläche liegt oder aber so, dass die Haupterstreckungsrichtung zumindest nahezu senkrecht zur Hautoberfläche liegt. Unter zumindest annährend linsenförmig wird in diesem Zusammenhang verstanden, dass es sich hierbei nicht zwangsläufig um die Idealform einer Linse handeln muss, sondern es sich vielmehr um einen räumlichen Körper handelt, der an seinen Außenflächen annähernd oval gerundet ist. Hierbei ist es durchaus denkbar, dass der Strahlungsbereich, der eine räumliche Ausdehnung annimmt, nicht symmetrisch oder gleichförmig ausgeführt ist, sondern insbesondere beispielsweise eine tropfenartige Form annimmt. In jedem Fall wird das Laserlicht derart gesteuert in die Haut bzw. das subkutane Gewebe eingebracht, dass ein räumlich ausgedehnter Bestrahlungsbereich in Abhängigkeit der zu behandelnden Zielstruktur gebildet wird.

Gemäß einer weiteren vorteilhaften Ausführungsform ist vorgesehen, dass die Laserlichtquelle und/oder das Lichtlenkmittel derart ansteuerbar sind, dass der Bereich, in dem eine gesteuerte Eigenschaftsveränderung realisiert wird, torusförmig ausgebildet ist. Wesentlich hierbei ist, dass im Inneren des dreidimensional ausgedehnten Bestrahlungsbereichs ein Raum verbleibt, in den zumindest nur sehr wenig Strahlungsenergie eingebracht wird, so dass es hier nicht zu einer nachhaltigen Eigenschaftsveränderung des Gewebes kommt.

Gemäß einer weiteren Ausführungsform der Erfindung sind eine erste sowie wenigstens eine zweite Laserlichtquelle vorgesehen. Auch in diesem Fall werden die wenigstens zwei Laserlichtquellen und/oder das zumindest eine Lichtlenkmittel derart angesteuert, dass Laserlichtstrahlung zumindest zeitweise auf unterschiedlichen Strahlengängen in den Bereich der Zielstruktur eingekoppelt wird, wobei in der Haut und/oder in subkutanem Gewebe ein Bestrahlungsbereich mit räumlicher Ausdehnung erzeugt wird. Wesentlich ist wiederum, dass weder in dem Bereich, in dem die Laserstrahlung auf die Haut auftritt, also insbesondere im Bereich der Hornschicht, als auch im Inneren der Haut bzw. des bestrahlten Gewebes nicht auf einen Punkt fokussiert wird, sondern stets ein Bereich mit räumlicher Ausdehnung so viel Strahlungsenergie absorbiert, dass es zu einer gezielten Gewebeveränderung kommt.

In einer besonderen Weiterbildung der Erfindung ist es denkbar, dass das Lichtlenkelement wenigstens ein von der Steuerung beeinflussbares Bewegungsmittel aufweist, über das zumindest die Laserlichtquelle bewegbar ist. Ebenso ist es grundsätzlich denkbar, dass die gesamte Bestrahlungsvorrichtung geregelt bewegt wird.
Gemäß dieser Ausführungsform ist somit wenigstens ein Bewegungsmittel, vorzugsweise in Form eines Elektromotors mit oder ohne Getriebe vorgesehen, so dass die Laserlichtquelle selbst und/oder wenigstens ein weiteres optisches Bauteil des Lichtlenkelementes derart bewegt wird, dass Laserlicht zumindest zeitweise über unterschiedliche Strahlengänge in die Zielstruktur eingestrahlt wird. Auf diese Weise wird wiederum sichergestellt, dass in der Haut oder dem zu behandelnden Gewebe ein räumlich ausgedehnter Bestrahlungsbereich, in dem eine gezielte Eigenschaftsveränderung des Gewebes oder der Haut hervorgerufen wird, realisiert wird. Auf besonders vorteilhafte Weise ist es denkbar eine Laserlichtquelle oder eine Anordnung wenigstens zweier Laserlichtquellen bewegbar, beispielsweise rotierend oder linear oszillierend, anzuordnen, so dass im Inneren der Haut oder des subkutanen Gewebes ein räumlich ausgedehnter Bestrahlungsbereich in dem des zu nachhaltigen Eigenschaftsveränderungen der Haut oder des Gewebes kommt, zu erzeugen. Gemäß einer ganz besonderen Ausführungsform ist es denkbar, eine Anordnung von Laserlichtquellen in einem Bestrahlungsgerät vorzusehen, wobei das Bestrahlungsgerät vorzugsweise als Handgerät, das von einer Person einfach getragen und manuell zu bedienen ist, ausgeführt ist. Das gemäß dieser Ausführungsform gestaltete Bestrahlungsgerät wird zur Behandlung auf die Haut des Patienten aufgesetzt oder über den zu behandelnden Bereich gehalten und eingeschaltet. Während der Behandlung wird die Anordnung von Laserlichtquellen automatisch gedreht, so dass im Bereich der Zielstruktur ein räumlich ausgedehnter Bestrahlungsbereich geschaffen wird. Sowohl die automatische Drehung als auch die Formung der Laserstrahlung wird von der Gerätesteuerung gesteuert. Gleichzeitig mit der Bestrahlung wird auf bevorzugte Weise der Oberflächenbereich der Haut gekühlt, um besonders zuverlässig eine nachhaltige Schädigung dieses Bereichs aufgrund der auftreffenden und hindurchtretenden Laserstrahlung zu vermeiden.

Generell ist gemäß einer besonders vorteilhaften Weiterbildung der Erfindung wenigstens ein Kühlelement vorgesehen, mit dem eine Hautoberfläche, insbesondere eine Oberfläche der Hornschicht, kühlbar ist. Durch das entsprechend vorgesehene Kühlelement wird sichergestellt, dass vor allem eine obere Hautschicht während der Behandlung der Zielstruktur mit Hilfe eines Lasers gekühlt wird, um so eine ungewünschte, nachhaltige Beeinträchtigung der oberen Hautschichten aufgrund der Laserbehandlung zu vermeiden. Das Kühlelement ist hierbei vorzugsweise derart ausgeführt, dass ein zu kühlender Bereich gezielt einstellbar ist, um einerseits die für die Kühlung benötigte Energie zu minimieren und andererseits eine zu starke Auskühlung der Haut, die wiederum zur Schädigung führen könnte, zu vermeiden. Hierbei ist es denkbar, dass sowohl die für die Kühlung verwendete Energie als auch der Hautbereich, der gekühlt werden soll, vom Anwender einstellbar ist.

In einer weiteren vorteilhaften Ausführungsform der Erfindung ist vorgesehen, dass in der Steuerung oder einem an die Steuerung gekoppelten Datenspeicher optische Eigenschaften wenigstens einer Hautschicht und/oder eines Gewebetyps hinterlegt sind. Auf vorteilhafte Weise ist es hierbei möglich, die Steuerung der Laserlichtquelle und/oder des Lichtlenkelementes auf der Grundlage von optischen Eigenschaften des bestrahlten Gewebes durchzuführen. Vorzugsweise sind sowohl optische Eigenschaften der durchstrahlten Haut- und/oder Gewebebereiche als auch der Bereiche, die die Zielstruktur bilden, in einem entsprechenden Datenspeicher abgelegt. Vorzugsweise werden als optische Eigenschaften insbesondere Berechnungsindizes, Absorptionsvermögen, Reflektivitäten und/oder Dichtewerte einzelner Haut- und/oder Gewebetypen in einem Datenspeicher abgelegt und sind so einer geeigneten Steuerung der Laserlichtquelle und/oder eines Lichtlenkelementes zu Grunde zu legen. Wesentlich hierbei ist, dass es aufgrund einer derart geeigneten Steuerung des Laserlichtes möglich ist, die unweigerlich stattfindenden Brechungen bzw. Teilreflexionen der Strahlung, die insbesondere am Übergang zwischen Haut- und Gewebetypen unterschiedlicher Dichte stattfinden, bei der Bestrahlung zu berücksichtigen und so auf ganz besonders geeignete Art die räumliche Ausdehnung eines Bestrahlungsbereiches zu erwirken.

Weiterhin bevorzugt ist vorgesehen, dass das wenigstens eine Lichtlenkelement zumindest einen Spiegel, einen Strahlteiler, ein optisches Filter, eine Blende, eine Linse, einen Spalt und/oder ein Prisma aufweist. Das Lichtlenkelement ist auch in diesem Fall derart ausgeführt, dass eine geeignete Strahlführung der Laserstrahlung realisiert wird. Hierbei ist es durchaus denkbar, dass das Lichtlenkelement sowohl über einen bevorzugt elektromotorischen Antrieb verfügt, durch den die Laserlichtquelle steuerbar bewegt wird und darüber hinaus wenigstens eines der vorgenannten optischen Bauteile vorgesehen ist. Selbstverständlich ist es ebenfalls denkbar, wenigstens eine Laserlichtquelle während der Bestrahlung in unveränderter Stellung zu belassen und wenigstens ein anderes Bauteil, insbesondere eine Linsenanordnung und/oder einen Reflektor derart geeignet zu bewegen, dass Laserlicht zumindest zeitweise über unterschiedliche Strahlengänge in die Zielstruktur eingestrahlt wird und hier ein räumlich ausgedehnter Bestrahlungsbereich erzeugt wird. In jedem Fall wird davon abgesehen, eine punktuelle Fokussierung der Strahlung vorzunehmen.

Gemäß einer besonderen Ausführungsform der Erfindung wird als Laserlichtquelle ein He: Ne-Laser, ein Nd:YAG-Laser und/oder ein Er:YAG-Laser verwendet. Grundsätzlich ist die Erfindung aber nicht auf den Einsatz eines speziellen Lasers als Lichtquelle beschränkt. Vielmehr wird in Abhängigkeit des zu behandelnden Gewebes bzw. der zu behandelnden Hauterkrankung ein Laser mit der jeweils benötigen Energie bzw. Lichtwellenlänge ausgesucht. Der entsprechende Laser wird wiederum mit geeigneten Lichtlenkelementen gekoppelt, so dass gezielt ein räumlicher Bestrahlungsbereich im Bereich der Zielstruktur erzeugt wird.

Neben einer Vorrichtung betrifft die Erfindung des Weiteren ein Verfahren zur Steuerung eines mit einer Laserlichtquelle bewirkten Energieeintrags in eine Zielstruktur. Die Zielstruktur befindet sich innerhalb menschlicher oder tierischer Haut oder Gewebe. In diesem Verfahren wird mittels einer Steuerung die Laserlichtquelle und/oder ein Lichtlenkmittel derart beeinflusst, dass eine Eigenschaftsveränderung der Haut und/oder des Gewebes zumindest nahezu ausschließlich im Bereich der Zielstruktur und zwar in einem dreidimensional ausgedehnten Bestrahlungsbereich realisiert wird.
Das erfindungsgemäße Steuerungsverfahren zeichnet sich dadurch aus, dass die Strahlengänge und/oder ein Pulsen der Laserlichtstrahlen derart variiert werden, dass ein die Eigenschaftsveränderung verursachender Energieeintrag in einem räumlich ausgedehnten Bereich, der zumindest nahezu ausschließlich innerhalb der Zielstruktur liegt, verursacht wird. Das erfindungsgemäße Steuerungsverfahren für eine Laserlichtquelle beruht somit wiederum auf dem wesentlichen Gedanken, dass Laserlicht auf unterschiedlichen Wegen in die Zielstruktur eingekoppelt wird und dennoch ein bestrahlter Bereich, der eine räumliche Ausdehnung aufweist, realisiert wird.

Weiterhin beruht ein Aspekt der Erfindung auf der Verwendung einer Mehrzahl von Laserstrahlen, die mit einer Vorrichtung, die wenigstens eines der zuvor beschriebenen erfindungswesentlichen technischen Merkmale aufweist, erzeugt wurden und einen räumlich ausgedehnten Bestrahlungsbereich innerhalb der Haut oder des subkutanen Gewebes schaffen, zur Behandlung von Akne, Hautverunreinigungen, Hämangiomen, Zellulitis, Hyperhidrose, Hautkrebs und/oder Hautfalten. Erfindungswesentlich ist somit die Verwendung eines mit der erfindungsgemäßen Vorrichtung hergestellten Bündels von Laserstrahlen, das einen räumlich ausgedehnten Bestrahlungsbereich innerhalb des zu behandelnden Gewebes und/oder der zu behandelnden Haut schafft.

Im Folgenden wird die Erfindung ohne Beschränkung des allgemeinen Erfindungsgedankens anhand von Ausführungsbeispielen unter Bezugnahme auf die Figuren näher beschrieben. Dabei zeigen:
- Figur 1:: schematische Darstellung einer Bestrahlungsvorrichtung mit Laserlichtquelle zur Schaffung eines Bestrahlungsbereichs mit räumlicher Ausdehnung,
- Figur 2:: Handstück mit Laserlichterzeugung sowie
- Figur 3:: Laserlichtquelle mit einem Lichtleitelement, das eine Strahlführung analog zu der eines Schwarzschild-Mikroskops ermöglicht.

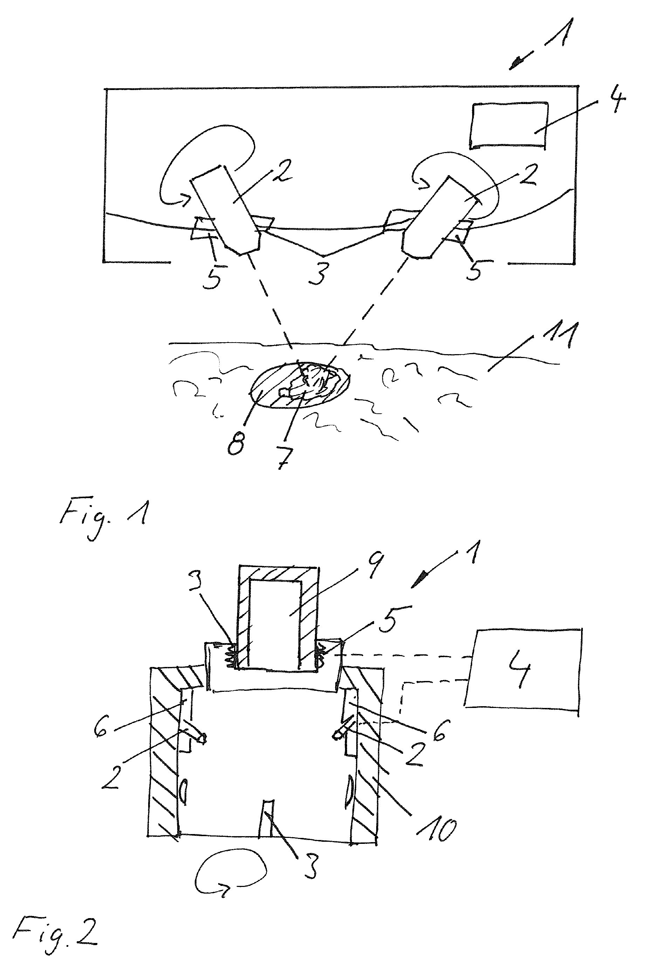

Figur 1 zeigt eine erfindungsgemäß ausgeführte Bestrahlungsvorrichtung 1 mit zwei Laserlichtquellen 2, die an einer geeigneten Halterung 6 fixiert sind, so dass eine Zielstruktur 7 innerhalb der Haut 11 oder des subkutanen Gewebes eines Patienten bestrahlt werden kann. Die beiden Laserlichtquellen 2 werden mit Hilfe einer Steuerung 4 gezielt angesteuert, so dass Laserstrahlen auf wenigstens zwei unterschiedlichen Wegen die Zielstruktur 7 innerhalb der Haut 11 oder des subkutanen Gewebes erreichen. Hierbei erfolgt in Abhängigkeit der geplanten Behandlung ein unterbrochener bzw. gepulster Betrieb der beiden Laser, wobei die Strahlung zumindest teilweise überlagert werden kann. Die Laserlichtquellen 2 werden stets derart angesteuert, dass in der Haut 11 oder dem Gewebe ein räumlich ausgedehnter Bestrahlungsbereich 8 erzeugt wird.

Gemäß der im Zusammenhang mit Figur 1 erläuterten Ausführungsform, hat der Bestrahlungsbereich 8, in dem gezielt eine nachhaltige Änderung der Haut 11 bzw. des Gewebes bewirkt wird, zumindest annähernd die Form einer Linse bzw. eines Reiskorns. Die Haupterstreckungsrichtung liegt hierbei nahezu parallel zur Hautoberfläche des Patienten. Selbstverständlich ist es denkbar, in Abhängigkeit der gewünschten Behandlung bzw. der Eigenschaften des zu behandelnden Gewebes, die Laser 2 derart anzusteuern, dass der Bestrahlungsbereich 8 eine andere Raumform annimmt, beispielsweise annähernd die Form eines Torus.

In der dargestellten Ausführungsform kann das Lichtlichtelement 3 in einfachster Form als Zeitschalter ausgeführt sein, so dass beispielsweise beide Laserlichtquellen 2 zu unterschiedlichen Zeitpunkten Licht in den Bereich der Zielstruktur 7 einstrahlen. Hierbei ist es weiterhin denkbar, dass anstelle des zweiten Lasers 2 entweder eine Mehrzahl von Lasern 2 vorgesehen ist und/oder ein weiteres Lichtlenkelement 3, insbesondere ein Reflektor, der derart ausgeführt und angeordnet ist, dass wenigstens zwei Laserlichtstrahlen auf unterschiedlichen Wegen in den Bestrahlungsbereich 8 mit räumlicher Ausdehnung, innerhalb dem die Zielstruktur 7 liegt, eingestrahlt werden.

Die Erzeugung eines gezielt festgelegten Bestrahlungsbereichs 8 mit räumlicher Ausdehnung hat einerseits den Vorteil, dass die Haut 11, insbesondere die äußere Hornschicht, auf die die Strahlung auftrifft, nicht nachhaltig geschädigt wird und andererseits auch im inneren der Haut 11 und/oder des bestrahlten Gewebes zumindest nahezu ausschließlich die zu behandelnde Haut 11 bzw. das Gewebe in der gewünschten Form geschädigt wird.

Um eine nachhaltige Beeinträchtigung der oberen Hautschichten zuverlässig zu vermeiden, ist des Weiteren ein Kühlelement 12 vorgesehen, durch das diese Hautschichten gekühlt werden. Die Kühlung erfolgt bevorzugt mit einer geeigneten Regelung, so dass gezielt in einem bestimmten Hautbereich eine Solltemperatur einstellbar ist. Alternativ oder in Ergänzung wird die Kühlung mit einem Kühlkissen und/oder einem Kühlspray bewirkt. Eine unerwünschte bzw. beeinträchtigende Erwärmung der Hautschichten aufgrund der Lasereinstrahlung wird hierdurch zuverlässig vermieden.

Um in einer Tiefe von etwa 3 mm eine Denaturierung von Collagen zu erreichen, werden Laserlichtquellen 2 verwendet, die eine gepulste Strahlung mit einer Wellenlänge von bevorzugt 1064 nm aussenden. Weiterhin werden die ausgesandten Lichtstrahlen entweder durch Bewegung zusätzlich vorgesehener Reflektoren 3 und/oder der Laserlichtquellen 2 derart in Bezug auf ihre Strahlengänge variiert, dass die gewünschte Gewebeveränderung, hier verbunden mit einer Collagendenaturierung, über einen ausgedehnten räumlichen Bestrahlungsbereich 8 geschaffen wird. Auf bevorzugte Weise sind für eine derartige Gestaltung der Bestrahlungsanordnung 1 Bewegungsmittel 5 mit einem Elektromotor vorgesehen, mit denen die Laserlichtquellen 2 gezielt bewegt werden. Eine gezielte Bewegung der Laserlichtquellen 2 wird durch die zentrale Steuerung 4 und auf der Grundlage von optischen Eigenschaften der be- und durchstrahlten Haut- und Gewebeschichten bewirkt. Für die Erzeugung des gewünschten räumlich ausgedehnten Bestrahlungsbereichs 8 werden optische Parameter, wie das Absorptionsvermögen, der Brechungsindex, der Reflexionsgrad und/oder die Dichte des jeweiligen Gewebes berücksichtigt.

Figur 2 zeigt ein Gerät 1 zur Durchführung von dermatologischen Behandlungen mittels Laserlicht. Das Bestrahlungsgerät 1 kann mit der Hand bedient und geführt werden und wird zur Behandlung über den zu bestrahlenden Bereich gehalten.
Das gezeigte Bestrahlungsgerät 1 weist im Wesentlichen einen Gehäusekörper 10, in dem eine Mehrzahl von Laserlichtquellen 2 angeordnet ist, sowie ein Handstück 9 auf, wobei die Anordnung mit den Laserlichtquellen 2 relativ zum Handstück 9 bewegbar, in diesem Fall drehbar ist.
Mit dem dargestellten Handbestrahlungsgerät 1 ist es möglich, auf einfache Weise eine Behandlung von kranker Haut oder subkutanem Gewebe durchzuführen. Innerhalb des Gerätekörpers 10 sind drei Laser 2 angeordnet, die über eine Linsenanordnung 3 Laserstrahlen auf unterschiedlichen Strahlengängen in den Bestrahlungsbereich 8 aussenden. In Abhängigkeit der geplanten Behandlung wird das Gerätegehäuse 10 mit den darin befindlichen Laserlichtquellen 2 unter Berücksichtigung von durch die Steuerung 4 erzeugten Steuersignalen, die auf einen Elektromotor mit Bewegungsmitteln 5 einwirken, relativ zum Handstück 9 gedreht. Der maximale Außendurchmesser des Handbestrahlungsgeräts 1 im Bereich des Gehäusekörpers 10 beträgt etwa 6 cm. Bei einer besonderen konstruktiven Gestaltung liegt dieser Durchmesser bei etwa 3,5 bis 5 cm.

In der Steuerung 4, in der Steuersignale zum Betrieb des Elektromotors 5 erzeugt werden, sind optische Eigenschaften des zu be- und durchstrahlenden Gewebes und der Haut 11 hinterlegt. Vor Behandlungsbeginn kann somit eine Zielstruktur 7 innerhalb der Haut 11 oder des subkutanen Gewebes lokalisiert und ein geeigneter dreidimensionaler Bestrahlungsraum 8 festgelegt werden. Im Weiteren werden für die Bestrahlung die festgelegten Strahlungsparameter der Laserstrahlung sowie die in der Steuerung 4 hinterlegten optischen Eigenschaften der durchstrahlten Bereiche einerseits und der Zielstruktur 7 andererseits berücksichtigt. Auf diese Weise wird sichergestellt, dass in der Haut 11 bzw. im subkutanen Gewebe ein räumlicher Bestrahlungsbereich 8 erzeugt wird, in dem die gewünschten Eigenschaftsveränderungen bewirkt werden.

Wesentlich an dem in Figur 2 gezeigten Handbestrahlungsgerät 1 ist vor allem, dass die im Inneren angeordneten Laserlichtquellen 2 relativ zur Zielstruktur 7 bewegbar sind, wobei in diesem Fall das Gehäuse 10 mit der Anordnung mehrerer Laserlichtquellen 2 gegenüber dem Handgriff 9 drehbar gelagert ist. Eine entsprechend geeignete Drehung wird mit Hilfe eines Elektromotors und der daran angebundenen Bewegungsmittel 5, die ebenfalls von der erfindungsgemäß ausgeführten Steuerung 4 angesteuert werden, realisiert. Während der Behandlung werden die Laser 2 derart bewegt, dass die einzelnen Laserstrahlen zunächst auf unterschiedliche äußere Hautschichten auftreffen und im Weiteren auf unterschiedlichen Strahlengängen durch die Hautschichten und das Gewebe bis in die Zielstruktur 7 gelangen. Innerhalb der Zielstruktur 7 findet eine Absorption Strahlungsenergie statt, so dass eine Temperaturerhöhung stattfindet, die zu den gewünschten Eigenschaftsveränderungen des Gewebes führt.

Gleichzeitig wird sichergestellt, dass außerhalb des Bestrahlungsbereichs 8, in dem sich die Zielstruktur 7 befindet, liegendes Gewebe sowie Hautschichten nicht nachhaltig durch die Laserbehandlung geschädigt werden. Hierbei werden über die Steuerung 4 sowohl die zeitliche Ansteuerung der einzelnen Laserlichtquellen 2, die durch eine Antriebseinheit 3 bewirkte Rotation der Halterung 6 mit den darin befindlichen Laserlichtquellen 2 sowie die Festlegung des räumlich ausgedehnten Bestrahlungsbereichs 8 realisiert. Zusätzlich sind auch in diesem Fall Mittel 12 zur Oberflächenkühlung der Hautschichten vorgesehen, auf die die Laserstrahlung zunächst auftrifft und die nicht einer Behandlung unterzogen werden sollen. Durch die zusätzliche Kühlung wird auf zuverlässige Weise sichergestellt, dass insbesondere im Oberflächenbereich der Haut keine unzulässige Erwärmung auftritt.

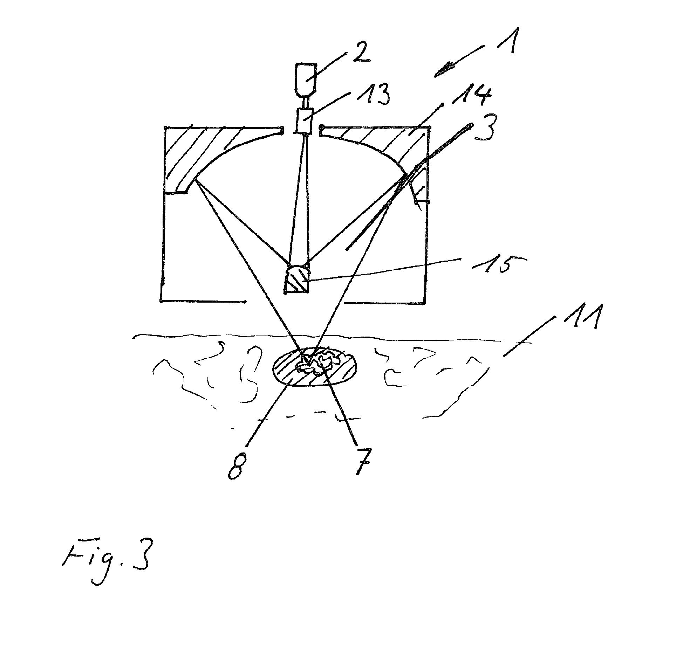

In Figur 3 wird ein weiteres Ausführungsbeispiel der Erfindung, durch das ein Bestrahlungsbereich 8 mit räumlicher Ausdehnung erzeugt wird, gezeigt. Wesentlich ist in diesem Fall, dass die von der wenigstens einen Laserlichtquelle 2 ausgesandte Strahlung mit Hilfe eines Schwarzschild-Objektivs auf den zu behandelnden Bereich, insbesondere in die Zielstruktur 7 gelenkt wird. Das in diesem Fall als Lichtlenkelement 3 verwendete Schwarzschild-Objektiv zeichnet sich vor allem dadurch aus, dass ein Lichtstrahl, der eine inhomogene Intensitätsverteilung mit höherer Intensität in der Strahlmitte als am Strahlrand aufweist, wie es beispielsweise bei Lichtstrahlen, die von Excimerlasern emittiert werden, der Fall ist, verkleinert abgebildet wird. Excimerlaser emittieren kohärente UV-Lichtstrahlen hoher Intensität und werden daher vielfach in der Medizin eingesetzt.

Von großer Bedeutung in einem Schwarzschild-Objektiv 3 sind zwei einander gegenüberliegend, zentrisch angeordneten Spiegel. Der Hauptspiegel 14 ist als Hohlspiegel konzipiert, dessen Spiegelfläche zur Hautoberfläche gerichtet ist. Der deutlich kleinere Auffangspiegel 15 liegt als Wölb- bzw. Konvexspiegel in objektnaher Position und seine Spiegelfläche zeigt zum Hauptspiegel 14.

Die von der Laserlichtquelle 2 emittierten Lichtstrahlen gelangen zunächst in eine Optik 13, durch die eine Intensitätsverteilung des Lichtstrahls vor Eintritt in das Schwarzschild-Objektiv 3 derart verändert wird, dass der Lichtstrahl in der Mitte eine geringere Intensität als in den Randbereichen hat. Die Optik 13 kann hierfür etwa über ein Biprisma und einen Homogenisierer verfügen. Anschließend werden die Lichtstrahlen mit Hilfe des Hauptspiegels 14 und des Auffangspiegels 15 auf den festgelegten Bestrahlungsbereich 8 fokussiert. Die wenigstens eine Laserlichtquelle 2 und/oder das in diesem Fall als Lichtlenkelement 3 vorgesehene Schwarzschild-Objektiv werden derart bewegt, dass wiederum ein räumlich ausgedehnter Bestrahlungsbereich 8 erzeugt wird, in dem sich die Zielstruktur 7 befindet. Ferner ist in Abhängigkeit der gewählten Behandlung ein Kühlelement vorgesehen, das die Hautoberfläche kühlt und so vor einer ungewünschten Schädigung durch die Laserbehandlung schützt.

### Bezugszeichenliste

### (Teil der Beschreibung)

- 1: Bestrahlungsanordnung
- 2: Laserlichtquelle
- 3: Lichtlenkelement
- 4: Steuerung
- 5: Bewegungsmittel
- 6: Halterung
- 7: Zielstruktur
- 8: Bestrahlungsbereich
- 9: Handstück
- 10: Gehäusekörper
- 11: Haut
- 12: Kühlelement
- 13: Optik
- 14: Hauptspiegel
- 15: Auffangspiegel

## Patentansprüche

1. Vorrichtung zur Bestrahlung einer Zielstruktur (7), die sich innerhalb von menschlicher oder tierischer Haut (11) oder Gewebe befindet, mit wenigstens einer Laserlichtquelle (2) und zumindest einem Lichtlenkmittel (3), die derart ausgeführt sind, dass Laserstrahlung über unterschiedliche Strahlengänge in die Zielstruktur (7) gelangt und aufgrund eines Energieeintrags in die Zielstruktur (7) zumindest bereichsweise eine Eigenschaftsveränderung der Zielstruktur (7) erfolgt,
**dadurch gekennzeichnet, dass** eine Steuerung (4) vorgesehen ist, so dass die Eigenschaftsveränderung gezielt in einem Bereich (8) mit räumlicher Ausdehnung realisierbar ist.

2. Vorrichtung nach Anspruch 1,
**dadurch gekennzeichnet, dass** die Laserlichtquelle (2) und/oder das Lichtlenkmittel (3) derart ansteuerbar sind, dass der Bereich (8), in dem eine gesteuerte Eigenschaftsveränderung realisiert wird, linsenförmig ausgebildet ist.

3. Vorrichtung nach Anspruch 1,
**dadurch gekennzeichnet, dass** die Laserlichtquelle (2) und/oder das Lichtlenkmittel (3) derart ansteuerbar sind, dass der Bereich (8), in dem eine gesteuerte Eigenschaftsveränderung realisiert wird, torusförmig ausgebildet ist.

4. Vorrichtung nach einem der Ansprüche 1 bis 3,
**dadurch gekennzeichnet, dass** eine erste und wenigstens eine zweie Laserlichtquelle (2) vorgesehen sind.

5. Vorrichtung nach einem der Ansprüche 1 bis 4,
**dadurch gekennzeichnet, dass** das Lichtlenkmittel (3) wenigstens ein von der Steuerung (4) beeinflussbares Bewegungsmittel (5) aufweist, über das zumindest die Laserlichtquelle (2) bewegbar ist.

6. Vorrichtung nach einem der Ansprüche 1 bis 5,
**dadurch gekennzeichnet, dass** zumindest ein Kühlelement (13) vorgesehen ist, mit dem eine Hautoberfläche, insbesondere eine Oberfläche der Hornschicht, kühlbar ist.

7. Vorrichtung nach einem der Ansprüche 1 bis 6,
**dadurch gekennzeichnet, dass** in der Steuerung (4) oder einem an die Steuerung (4) gekoppelten Datenspeicher optische Eigenschaften wenigstens einer Hautschicht und/oder eines Gewebetyps hinterlegt sind.

8. Vorrichtung nach Anspruch 7,
**dadurch gekennzeichnet, dass** als optische Eigenschaft wenigstens ein Wert für einen Brechungsindex und/oder für ein Absorptionsvermögen hinterlegt ist.

9. Vorrichtung nach einem der Ansprüche 1 bis 8,
**dadurch gekennzeichnet, dass** die Steuerung (4) unter Berücksichtigung optischer Eigenschaften wenigstens einer Hautschicht und/oder eines Gewebetyps auf die Laserlichtquelle (2) und/oder das Lichtlenkmittel (3) einwirkt.

10. Vorrichtung nach einem der Ansprüche 1 bis 9,
**dadurch gekennzeichnet, dass** das Lichtlenkelement (3) wenigstens einen Spiegel, einen Strahlteiler, ein optisches Filter, eine Blende, eine Linse, einen Spalt und/oder ein Prisma aufweist.

11. Vorrichtung nach einem der Ansprüche 1 bis 10,
**dadurch gekennzeichnet, dass** die Laserlichtquelle (2) einen He-Ne-Laser, einen Nd-YAG-Laser und/oder einen Er-YAG-Laser aufweist.

12. Verfahren zur Steuerung eines mit einer Laserlichtquelle bewirkten Energieeintrags in eine Zielstruktur, die sich innerhalb von menschlicher oder tierischer Haut oder Gewebe befindet, bei dem mittels einer Steuerung die Laserlichtquelle und/oder ein Lichtlenkmittel derart beeinflusst werden, dass eine Eigenschaftsveränderung der Haut und/oder des Gewebes zumindest nahezu ausschließlich im Bereich der Zielstruktur realisiert wird,
**dadurch gekennzeichnet, dass** die Strahlengänge der Laserlichtstrahlung derart variiert werden, dass ein die Eigenschaftsveränderung verursachender Energieeintrag in einem räumlich ausgedehnten Bereich, der zumindest nahezu ausschließlich innerhalb der Zielstruktur liegt, verursacht wird.

13. Verwendung eines mit einer Vorrichtung gemäß einem der Ansprüche 1 bis 11 erzeugten Laserstrahls zur Behandlung von Akne, Hautverunreinigungen, Hämangiomen, Zellulitis, Hyperhidrose, Hautkrebs, Hautfalten, Varicosis, Bandscheibenprolaps und/oder Fett.
